# EUROPEAN PATENT APPLICATION

(11) **EP 4 039 814 A1**
(43) Date of publication of application: **10.08.2022**
(21) Application number: 20872500.2
(22) Date of filing: 02.10.2020
(51) Int. Cl.: C12P 19/04, C12N 5/10, C12N 15/12, C12N 15/54, C12N 15/63

(54) **METHOD FOR PRODUCING HEPARIN-LIKE SUBSTANCE, RECOMBINANT CELLS AND METHOD FOR PRODUCING SAME**

(30) Priority: 02.10.2019 JP 2019182467
(71) Applicant: Kyushu University, National University Corporation, Nishi-ku Fukuoka-shi Fukuoka 819-0395 (JP); National University Corporation Chiba University, Chiba-shi, Chiba 263-8522 (JP); Kyowa Hakko Bio Co., Ltd., Tokyo 100-8185 (JP)
(72) Inventor: KAMIHIRA Masamichi, Fukuoka-shi, Fukuoka 819-0395 (JP); KAWABE Yoshinori, Fukuoka-shi, Fukuoka 819-0395 (JP); SAGAWA Kosuke, Fukuoka-shi, Fukuoka 819-0395 (JP); TOIDA Toshihiko, Chiba-shi, Chiba 260-8675 (JP); KOIZUMI Satoshi, Tokyo 100-8185 (JP)
(74) Representative: Vossius & Partner Patentanwälte Rechtsanwälte mbB
(86) International application number: PCT/JP2020/037626
(87) International publication number: WO 2021/066167

(57) **Abstract**

An object of the present invention is to provide a method for efficiently producing a heparin-like substance without using an animal-derived tissue. The present invention relates to a method for producing a heparin-like substance and the like, the method comprising: (1) preparing a mammalian cell that produces a heparin-like substance, (2) preparing a recombinant cell in which a gene that encodes an extracellular domain of syndecan is introduced into the mammalian cell that produces a heparin-like substance and is prepared in step (1), and (3) culturing the recombinant cell prepared in step (2) in a medium and collecting the heparin-like substance from the resulting culture supernatant.

SEQUENCE LISTING

<110>  KYUSHU UNIVERSITY, NATIONAL UNIVERSITY CORPORATION
       NATIONAL UNIVERSITY CORPORATION CHIBA UNIVERSITY
       KYOWA HAKKO BIO CO., LTD.

<120>  The method for producing heparin-like substance, the recombinant
       cell and the method for producing thereof

<130>  W530034

<150>  JP2019-182467
<151>  2019-10-02

<160>  7

<170>  PatentIn version 3.5

<210>  1
<211>  229
<212>  PRT
<213>  Homo sapiens

<400>  1

Met Arg Arg Ala Ala Leu Trp Leu Trp Leu Cys Ala Leu Ala Leu Ser
1               5                   10                  15

Leu Gln Pro Ala Leu Pro Gln Ile Val Ala Thr Asn Leu Pro Pro Glu
            20                  25                  30

Asp Gln Asp Gly Ser Gly Asp Asp Ser Asp Asn Phe Ser Gly Ser Gly
        35                  40                  45

Ala Gly Ala Leu Gln Asp Ile Thr Leu Ser Gln Gln Thr Pro Ser Thr
    50                  55                  60

Trp Lys Asp Thr Gln Leu Leu Thr Ala Ile Pro Thr Ser Pro Glu Pro
65                  70                  75                  80

Thr Gly Leu Glu Ala Thr Ala Ala Ser Thr Ser Thr Leu Pro Ala Gly
                85                  90                  95

Glu Gly Pro Lys Glu Gly Glu Ala Val Val Leu Pro Glu Val Glu Pro
            100                 105                 110

Gly Leu Thr Ala Arg Glu Gln Glu Ala Thr Pro Arg Pro Arg Glu Thr
        115                 120                 125

Thr Gln Leu Pro Thr Thr His Gln Ala Ser Thr Thr Thr Ala Thr Thr
    130                 135                 140

Ala Gln Glu Pro Ala Thr Ser His Pro His Arg Asp Met Gln Pro Gly
145                 150                 155                 160

His His Glu Thr Ser Thr Pro Ala Gly Pro Ser Gln Ala Asp Leu His
                165                 170                 175

Thr Pro His Thr Glu Asp Gly Gly Pro Ser Ala Thr Glu Arg Ala Ala
            180                 185                 190

Glu Asp Gly Ala Ser Ser Gln Leu Pro Ala Ala Glu Gly Ser Gly Glu
        195                 200                 205

Gln Asp Phe Thr Phe Glu Thr Ser Gly Glu Asn Thr Ala Val Val Ala
    210                 215                 220

Val Glu Pro Asp Arg
225

<210>  2
<211>  883
<212>  PRT
<213>  Homo sapiens

<400>  2

Met Leu Gln Leu Trp Lys Val Val Arg Pro Ala Arg Gln Leu Glu Leu
1               5                   10                  15

His Arg Leu Ile Leu Leu Leu Ile Ala Phe Ser Leu Gly Ser Met Gly
            20                  25                  30

Phe Leu Ala Tyr Tyr Val Ser Thr Ser Pro Lys Ala Lys Glu Pro Leu
        35                  40                  45

Pro Leu Pro Leu Gly Asp Cys Ser Ser Gly Gly Ala Ala Gly Pro Gly
    50                  55                  60

Pro Ala Arg Pro Pro Val Pro Pro Arg Pro Pro Arg Pro Pro Glu Thr
65                  70                  75                  80

Ala Arg Thr Glu Pro Val Val Leu Val Phe Val Glu Ser Ala Tyr Ser
                85                  90                  95

Gln Leu Gly Gln Glu Ile Val Ala Ile Leu Glu Ser Ser Arg Phe Arg
            100                 105                 110

Tyr Ser Thr Glu Leu Ala Pro Gly Arg Gly Asp Met Pro Thr Leu Thr
        115                 120                 125

Asp Asn Thr His Gly Arg Tyr Val Leu Val Ile Tyr Glu Asn Leu Leu
    130                 135                 140

Lys Tyr Val Asn Leu Asp Ala Trp Ser Arg Glu Leu Leu Asp Arg Tyr
145                 150                 155                 160

Cys Val Glu Tyr Gly Val Gly Ile Ile Gly Phe Phe Arg Ala His Glu
                165                 170                 175

His Ser Leu Leu Ser Ala Gln Leu Lys Gly Phe Pro Leu Phe Leu His
            180                 185                 190

Ser Asn Leu Gly Leu Arg Asp Tyr Gln Val Asn Pro Ser Ala Pro Leu
        195                 200                 205

Leu His Leu Thr Arg Pro Ser Arg Leu Glu Pro Gly Pro Leu Pro Gly
    210                 215                 220

Asp Asp Trp Thr Ile Phe Gln Ser Asn His Ser Thr Tyr Glu Pro Val
225                 230                 235                 240

Leu Leu Ala Ser Leu Arg Pro Ala Glu Pro Ala Val Pro Gly Pro Val
                245                 250                 255

Leu Arg Arg Ala Arg Leu Pro Thr Val Val Gln Asp Leu Gly Leu His
            260                 265                 270

Asp Gly Ile Gln Arg Val Leu Phe Gly His Gly Leu Ser Phe Trp Leu
        275                 280                 285

His Lys Leu Ile Phe Val Asp Ala Val Ala Tyr Leu Thr Gly Lys Arg
    290                 295                 300

Leu Cys Leu Asp Leu Asp Arg Tyr Ile Leu Val Asp Ile Asp Asp Ile
305                 310                 315                 320

Phe Val Gly Lys Glu Gly Thr Arg Met Lys Val Ala Asp Val Glu Ala
                325                 330                 335

Leu Leu Thr Thr Gln Asn Lys Leu Arg Thr Leu Val Pro Asn Phe Thr
            340                 345                 350

Phe Asn Leu Gly Phe Ser Gly Lys Phe Tyr His Thr Gly Thr Glu Glu
        355                 360                 365

Glu Asp Ala Gly Asp Asp Met Leu Leu Lys His Arg Lys Glu Phe Trp
    370                 375                 380

Trp Phe Pro His Met Trp Ser His Met Gln Pro His Leu Phe His Asn

385                 390                 395                 400

Arg Ser Val Leu Ala Asp Gln Met Arg Leu Asn Lys Gln Phe Ala Leu
                405                 410                 415

Glu His Gly Ile Pro Thr Asp Leu Gly Tyr Ala Val Ala Pro His His
            420                 425                 430

Ser Gly Val Tyr Pro Ile His Thr Gln Leu Tyr Glu Ala Trp Lys Ser
        435                 440                 445

Val Trp Gly Ile Gln Val Thr Ser Thr Glu Glu Tyr Pro His Leu Arg
    450                 455                 460

Pro Ala Arg Tyr Arg Arg Gly Phe Ile His Asn Gly Ile Met Val Leu
465                 470                 475                 480

Pro Arg Gln Thr Cys Gly Leu Phe Thr His Thr Ile Phe Tyr Asn Glu
                485                 490                 495

Tyr Pro Gly Gly Ser Arg Glu Leu Asp Arg Ser Ile Arg Gly Gly Glu
            500                 505                 510

Leu Phe Leu Thr Val Leu Leu Asn Pro Ile Ser Ile Phe Met Thr His
        515                 520                 525

Leu Ser Asn Tyr Gly Asn Asp Arg Leu Gly Leu Tyr Thr Phe Glu Ser
    530                 535                 540

Leu Val Arg Phe Leu Gln Cys Trp Thr Arg Leu Arg Leu Gln Thr Leu
545                 550                 555                 560

Pro Pro Val Pro Leu Ala Gln Lys Tyr Phe Glu Leu Phe Pro Gln Glu
                565                 570                 575

Arg Ser Pro Leu Trp Gln Asn Pro Cys Asp Asp Lys Arg His Lys Asp
            580                 585                 590

Ile Trp Ser Lys Glu Lys Thr Cys Asp Arg Leu Pro Lys Phe Leu Ile
        595                 600                 605

Val Gly Pro Gln Lys Thr Gly Thr Thr Ala Ile His Phe Phe Leu Ser
    610                 615                 620

Leu His Pro Ala Val Thr Ser Ser Phe Pro Ser Pro Ser Thr Phe Glu
625                 630                 635                 640

Glu Ile Gln Phe Phe Asn Ser Pro Asn Tyr His Lys Gly Ile Asp Trp
                645                 650                 655

Tyr Met Asp Phe Phe Pro Val Pro Ser Asn Ala Ser Thr Asp Phe Leu
            660                 665                 670

Phe Glu Lys Ser Ala Thr Tyr Phe Asp Ser Glu Val Val Pro Arg Arg
        675                 680                 685

Gly Ala Ala Leu Leu Pro Arg Ala Lys Ile Ile Thr Val Leu Thr Asn
    690                 695                 700

Pro Ala Asp Arg Ala Tyr Ser Trp Tyr Gln His Gln Arg Ala His Gly
705                 710                 715                 720

Asp Pro Val Ala Leu Asn Tyr Thr Phe Tyr Gln Val Ile Ser Ala Ser
                725                 730                 735

Ser Gln Thr Pro Leu Ala Leu Arg Ser Leu Gln Asn Arg Cys Leu Val
            740                 745                 750

Pro Gly Tyr Tyr Ser Thr His Leu Gln Arg Trp Leu Thr Tyr Tyr Pro
        755                 760                 765

Ser Gly Gln Leu Leu Ile Val Asp Gly Gln Glu Leu Arg Thr Asn Pro
    770                 775                 780

Ala Ala Ser Met Glu Ser Ile Gln Lys Phe Leu Gly Ile Thr Pro Phe
785                 790                 795                 800

Leu Asn Tyr Thr Arg Thr Leu Arg Phe Asp Asp Asp Lys Gly Phe Trp
                805                 810                 815

Cys Gln Gly Leu Glu Gly Gly Lys Thr Arg Cys Leu Gly Arg Ser Lys
            820                 825                 830

Gly Arg Arg Tyr Pro Asp Met Asp Thr Glu Ser Arg Leu Phe Leu Thr
        835                 840                 845

Asp Phe Phe Arg Asn His Asn Leu Glu Leu Ser Lys Leu Leu Ser Arg
    850                 855                 860

Leu Gly Gln Pro Val Pro Ser Trp Leu Arg Glu Glu Leu Gln His Ser
865                 870                 875                 880

Ser Leu Gly

<210>  3
<211>  311
<212>  PRT
<213>  Homo sapiens

<400>  3

<210>  4
<211>  2652
<212>  DNA
<213>  Homo sapiens

<400>  4
atgctccagt tgtggaaggt ggtacgccca gctcggcagc tggaactgca ccgcctcata      60

ctgctgctga tcgctttcag cctgggctcc atgggcttcc tggcttatta tgtgtccacc     120

agccctaagg ccaaggaacc cttgcccctg cccttgggag actgcagcag cggtggggca     180

gctggtcctg gccctgcacg gcctccagtt ccacctcggc cccccaggcc tccagagaca     240

gctcgaactg aacccgtggt ccttgtgttt gtggagagtg catactcaca gctggggcag     300

gaaattgtgg ccatcctgga gtctagtcgt tttcgttata gcactgagtt ggcacctggc     360

cgaggggaca tgcccacatt gactgataat acccatggcc gctatgtctt ggtcatttat     420

gagaacctgc tcaagtatgt caacctggat gcctggagtc gggaactgct agaccggtac     480

tgcgtggagt atggtgtggg catcattggc tttttccgag cccacgagca cagcctactg     540

agcgcccagc tcaagggctt tccccttttt ttacactcaa acttggggct ccgggactac     600

caagtgaatc cttctgcccc gctactgcat ctcacacgcc ccagccgcct agaaccaggg     660

ccactgcctg gtgatgactg gaccatcttc caatccaatc atagtacata tgaaccagtg     720

cttcttgcca gccttcggcc agctgagccc gcagtgccag gaccagttct tcgtcgggcc     780

cggcttccca ctgtggtaca ggacctgggg cttcatgatg gcatccagcg ggtgctcttt     840

ggacatggcc tttccttctg gctccacaaa cttatcttcg ttgatgctgt tgcatacctc     900

actggcaagc gcctctgcct ggaccttgac cgctacatct tggtagacat cgatgacatc     960

tttgtgggca aggaagggac ccgcatgaag gtggctgatg ttgaggctct gttgaccacc 1020

cagaacaaac tcaggacctt agttcccaac ttcaccttca acttgggctt ctcgggcaag 1080

ttctatcata ctgggacaga ggaggaggat gcaggggacg acatgctgct gaagcaccgc 1140

aaagagttct ggtggttccc ccacatgtgg agccacatgc agccacacct gttccacaat 1200

cgctccgtgc tggctgacca gatgaggctc aacaaacagt ttgctctgga gcatgggatt 1260

cccacggacc tggggtatgc tgtggccccc caccactcgg gtgtgtaccc catccacacg 1320

cagctctatg aggcctggaa atccgtgtgg ggcatccagg tgaccagcac tgaggagtat 1380

ccccatctcc gccctgcccg ctaccgccgt ggcttcattc acaatggcat tatggtgctg 1440

ccccggcaga catgtggcct cttcactcac acaatcttct ataatgagta tcctggaggc 1500

tctcgtgaac tagaccggag catccgaggt ggagagctct ttctgacagt gctgcttaat 1560

ccgatcagca tctttatgac ccatctgtcc aattatggaa atgaccggct gggcctatac 1620

acctttgaga gcttggtgcg cttcctccag tgttggacac ggctgcgcct acagaccctt 1680

cctcctgtcc cacttgcaca gaagtacttt gaacttttcc ctcaggagcg aagccccctt 1740

tggcagaatc cctgtgatga caagaggcac aaagatatct ggtccaagga gaaaacctgt 1800

gatcgtctcc cgaagttcct cattgtggga ccccagaaaa cagggactac agctattcac 1860

ttcttcctga gcctgcaccc agctgtaact agcagcttcc ctagccccag cacatttgag 1920

gagattcagt tcttcaacag ccctaattac cacaagggta ttgactggta catggatttc 1980

ttccctgttc cttccaatgc cagcactgat ttcctatttg aaaaaagtgc cacctacttt 2040

gactctgaag ttgtaccacg gcggggggct gccctcctgc cacgagccaa gatcatcaca 2100

gtgctcacca accctgctga cagggcctac tcctggtacc agcatcagcg agcccatgga 2160

gacccagttg ctctgaacta taccttctat caggtgattt cagcctcctc ccagacccct 2220

ctggcactac gctccctgca gaaccgctgt cttgtccctg gctactattc tacccatcta 2280

caacgctggc tgacttacta cccctctgga cagttgctga ttgtggatgg gcaagagctg 2340

cgtaccaacc cagcagcctc aatggagagc atccagaagt tcctgggtat cacacccttt 2400

ctgaactaca cacggaccct caggtttgat gatgataagg gattttggtg ccagggactt 2460

gaaggtggta agactcgctg tctaggccgg agcaaaggcc ggaggtatcc agatatggac 2520

actgagtccc gtcttttcct tacggatttt ttccggaacc ataatttgga gttgtcgaag 2580

ctgctgagcc ggcttggaca gccagtgccc tcgtggcttc gggaagaact gcagcattcc 2640

agtctgggct ga 2652

<210> 5
<211> 936
<212> DNA
<213> Mus musculus

<400>  5
atgaccttgc tgctcctggg tgcggtgctg ctggtggccc agccccagct tgtgcattcc      60

cacccggctg ctcctggccc ggggctcaaa cagcaggagc ttctgaggaa ggtgattatt     120

ctcccagagg acaccggaga aggcacagca tccaatggtt ccacacagca gctgccacag     180

accatcatca ttggggtgcg caagggtggt acccgagccc tgctagagat gctcagcctg     240

catcctgatg ttgctgcagc tgaaaacgag gtccatttct ttgactggga ggagcattac     300

agccaaggcc tgggctggta cctcacccag atgcccttct cctcccctca ccagctcacc     360

gtggagaaga cacccgccta tttcacttcg cccaaagtgc ctgagagaat ccacagcatg     420

aaccccacca tccgcctgct gcttatcctg agggacccat cagagcgcgt gctgtccgac     480

tacacccagg tgttgtacaa ccaccttcag aagcacaagc cctatccacc cattgaggac     540

ctcctaatgc gggacggtcg gctgaacctg gactacaagg ctctcaaccg cagcctgtac     600

catgcacaca tgctgaactg gctgcgtttt ttcccgttgg gccacatcca cattgtggat     660

ggcgaccgcc tcatcagaga ccctttccct gagatccaga aggtcgaaag attcctgaag     720

ctttctccac agatcaacgc ctcgaacttc tactttaaca aaaccaaggg cttctactgc     780

ctgcgggaca gtggcaagga ccgctgctta cacgagtcca aaggccgggc gcacccccag     840

gtggatccca aactacttga taaactgcac gaatactttc atgagccaaa taagaaattt     900

ttcaagctcg tgggcagaac attcgactgg cactga                               936


<210>  6
<211>  687
<212>  DNA
<213>  Homo sapiens

<400>  6
atgaggcgcg cggcgctctg gctctggctg tgcgcgctgg cgctgagcct gcagccggcc      60

ctgccgcaaa ttgtggctac taatttgccc cctgaagatc aagatggctc tggggatgac     120

tctgacaact tctccggctc aggtgcaggt gctttgcaag atatcacctt gtcacagcag     180

accccctcca cttggaagga cacgcagctc ctgacggcta ttcccacgtc tccagaaccc     240

accggcctgg aggctacagc tgcctccacc tccaccctgc cggctggaga ggggcccaag     300

gagggagagg ctgtagtcct gccagaagtg gagcctggcc tcaccgcccg ggagcaggag     360

gccacccccc gacccaggga gaccacacag ctcccgacca ctcatcaggc ctcaacgacc     420

acagccacca cggcccagga gcccgccacc tcccaccccc acagggacat gcagcctggc     480

caccatgaga cctcaacccc tgcaggaccc agccaagctg accttcacac tccccacaca     540

gaggatggag gtccttctgc caccgagagg gctgctgagg atggagcctc cagtcagctc     600

ccagcagcag agggctctgg ggagcaggac ttcacctttg aaacctcggg ggagaatacg     660

gctgtagtgg ccgtggagcc tgaccgc 687

<210> 7
<211> 477
<212> DNA
<213> Homo sapiens

<400> 7
atgatgcaga agctactcaa atgcagtcgg cttgtcctgg ctcttgccct catcctggtt 60

ctggaatcct cagttcaagg ttatcctacg cggagagcca ggtaccaatg ggtgcgctgc 120

aatccagaca gtaattctgc aaactgcctt gaagaaaaag gaccaatgtt cgaactactt 180

ccaggtgaat ccaacaagat cccccgtctg aggactgacc tttttccaaa gacgagaatc 240

caggacttga atcgtatctt cccactttct gaggactact ctggatcagg cttcggctcc 300

ggctccggct ctggatcagg atctgggagt ggcttcctaa cggaaatgga acaggattac 360

caactagtag acgaaagtga tgctttccat gacaacctta ggtctcttga caggaatctg 420

ccctcagaca gccaggactt gggtcaacat ggattagaag aggattttat gttataa 477

## Description

### Technical Field

The present invention relates to a method for producing a heparin-like substance, a recombinant cell, and a method for producing the same.

### Background Art

Heparin is an essential drug that is used in medical practice such as artificial dialysis and extracorporeal circulation as an anticoagulant. Heparin is used in an injectable form, for example, as a prophylactic or therapeutic agent for thromboembolism. Heparin is also used to form an anticoagulant surface on various experimental and medical devices such as a kidney dialysis machine.

Heparin is a polysaccharide that is one of four major subfamilies of glycosaminoglycans (hereinafter also abbreviated as GAG). GAG is a long polysaccharide composed of a repetitive disaccharide unit. GAG is mainly classified into four subfamilies: (1) heparan sulfate (hereinafter also abbreviated as HS)/heparin, (2) keratan sulfate (KS), (3) chondroitin/dermatan sulfate (CS/DS), and (4) hyaluronic acid or hyaluronan (HA).

The subfamilies of GAG differ from each other in a monosaccharide component, a glycosidic linkage, and a sugar position and degree. Except for HA, GAG is covalently bound to a core protein, and GAG covalently bound to the core protein is known as proteoglycan.

Heparin and HS are heterogeneous hybrids of acidic mucopolysaccharides, and heparin is a type of HS. Heparin/HS is a linear polysaccharide comprising a disaccharide of uronic acid (β-D-glucuronic acid and α-L-iduronic acid) and glucosamine (DN-acetylglucosamine and DN-glucosamine sulfate) as one unit, those disaccharides being linked by tens to hundreds of α or β-1,4 linkages. A molecular weight varies from 3 kDa to 30 kDa depending on a difference in a chain length, with an average of 12 kDa to 15 kDa. Examples of modification of heparin/HS include O-sulfation of uronic acid at 2-position, O-sulfation of glucosamine at 3-position and 6-position, and N-sulfation of an amino group at 2-position.

Almost all mammalian cells produce GAGs, which are incorporated into proteoglycans in cell-associated sugar chains present in an extracellular matrix to define tissue morphology and function. HS regulates growth and generation of cells by regulating a growth factor such as a fibroblast growth factor (FGF) family, a platelet-derived growth factor (PDGF), and a vascular endothelial cell growth factor (VEGF). On the other hand, heparin is mainly produced in mast cells.

Heparin used in pharmaceutical products and the like is mainly extracted and purified from a small intestine of a pig or a lung of a bovine. However, heparin obtained from these supply sources has been associated with several adverse events due to contamination with persulfated chondroitin sulphate (Non-Patent Document 1). In addition, the isolation of heparin from animal tissues has a risk of propagation of pathogens such as viruses, bacteria, and transmissible spongiform encephalopathy from animals to humans. Therefore, development of a new technology for producing heparin without using an animal-derived tissue is desired. Although heparin can be synthesized by a chemical or chemoenzymatic method, it is difficult to produce a heterogeneous structural population present in animal-derived heparin.

On the other hand, syndecan is a family of four proteoglycans on a cell surface with a retained plasma membrane domain and a cytoplasmic domain. Covalently bound glycosaminoglycan chains are present in an extracellular domain. The covalently bound glycosaminoglycan chains are mainly heparan sulfate (HS), and also include chondroitin sulfate (CS) (Non-Patent Document 2).

### Related Art

### Patent Document

### Non-Patent Document

[Non-Patent Document 1] Guerrini et al, Nat. Biotechnol., 2008, 26, pp.669-675
[Non-Patent Document 2] Trends in Glycoscience and Glycotechnology, 1993, Vol.5, No.22, pp. 107-120

### Disclosure of Invention

### Technical Problem

### Problems to Be Solved by the Invention

As described above, development of a new technology for producing heparin without using an animal-derived tissue is desired. The present inventors have attempted to produce a heparin-like substance using a mammalian cell that produces a heparin-like substance, and found that proteoglycan containing a heparin-like substance produced from the mammalian cell adheres to a cell membrane and is less likely to be secreted into a culture supernatant. Therefore, a complicated purification process is required, which is a major problem in industrial production of a heparin-like substance. Accordingly, an object of the present invention is to provide a production method for efficiently producing a heparin-like substance without using an animal-derived tissue.

### Means for Solving the Problems

The present inventors have found that the above problems can be solved by introducing a gene that encodes an extracellular domain of syndecan into a mammalian cell that produces a heparin-like substance, and have completed the present invention.

The present invention is as follows.
1. A method for producing a heparin-like substance, the method comprising the following steps (1) to (3):
   (1) preparing a mammalian cell that produces a heparin-like substance;
   (2) preparing a recombinant cell in which a gene that encodes an extracellular domain of syndecan (hereinafter abbreviated as SDC) is introduced into the mammalian cell that produces a heparin-like substance and is prepared in step (1); and
   (3) culturing the recombinant cell prepared in step (2) in a medium, and collecting a heparin-like substance from a resulting culture supernatant.
2. The production method according to 1, in which the mammalian cell that produces a heparin-like substance is a cell into which at least one of a gene that encodes bifunctional heparan sulfate ester N-deacetylase/N-sulfate transferase (hereinafter abbreviated as NDST2) and a gene that encodes heparan sulfate ester glucosamine 3-sulfate transferase 1 (hereinafter abbreviated as Hs3st1) is introduced.
3. The production method according to 1 or 2, in which the mammalian cell is a Chinese hamster-derived ovary (hereinafter abbreviated as CHO) cell.
4. The production method according to 2 or 3, in which NDST2 is a protein comprising any one of the following amino acid sequences (1a) to (1c) and having a function of NDST2:
   (1a) the amino acid sequence of SEQ ID NO: 2,
   (1b) an amino acid sequence in which one to several amino acids are deleted, substituted or added in the amino acid sequence of SEQ ID NO: 2, and
   (1c) an amino acid sequence having 80% or more homology with the amino acid sequence of SEQ ID NO: 2.
5. The production method according to any one of 2 to 4, in which Hs3st1 is a protein comprising any one of the following amino acid sequences (2a) to (2c) and having a function of Hs3st1:
   (2a) the amino acid sequence of SEQ ID NO: 3,
   (2b) an amino acid sequence in which one to several amino acids are deleted, substituted or added in the amino acid sequence of SEQ ID NO: 3, and
   (2c) an amino acid sequence having 80% or more homology with the amino acid sequence of SEQ ID NO: 3.
6. The production method according to any one of 1 to 5, in which the extracellular domain of SDC is a protein comprising any one of the following amino acid sequences (3a) to (3c) and having a function of the extracellular domain of SDC:
   (3a) the amino acid sequence of SEQ ID NO: 1,
   (3b) an amino acid sequence in which one to several amino acids are deleted, substituted or added in the amino acid sequence of SEQ ID NO: 1, and
   (3c) an amino acid sequence having 80% or more homology with the amino acid sequence of SEQ ID NO: 1.
7. The production method according to any one of 2 to 6, in which NDST2 and Hs3st1 are derived from a mouse or a human.
8. The production method according to any one of 2 to 7, in which the gene that encodes NDST2 comprises a base sequence that is any one of the following base sequences (1A) to (1D) and encodes an amino acid sequence of a protein having the function of NDST2:
   (1A) the base sequence of SEQ ID NO: 4,
   (1B) a base sequence in which one to several bases are deleted, substituted or added in the base sequence of SEQ ID NO: 4,
   (1C) a base sequence which hybridizes under stringent conditions with a base sequence complementary to the base sequence of SEQ ID NO: 4, and
   (1D) a base sequence having 80% or more homology with the base sequence of SEQ ID NO: 4.
9. The production method according to any one of 2 to 8, in which the gene that encodes Hs3st1 comprises a base sequence that is any one of the following base sequences (2A) to (2D) and encodes an amino acid sequence of a protein having a function of Hs3st1:
   (2A) a base sequence of SEQ ID NO: 5,
   (2B) a base sequence in which one to several bases are deleted, substituted or added in the base sequence of SEQ ID NO: 5,
   (2C) a base sequence which hybridizes under stringent conditions with a base sequence complementary to the base sequence of SEQ ID NO: 5, and
   (2D) a base sequence having 80% or more homology with the base sequence of SEQ ID NO: 5.
10. The production method according to any one of 1 to 9, in which the gene that encodes an extracellular domain of SDC is any one of the following base sequences (3A) to (3D) and comprises a base sequence that encodes an amino acid sequence of a protein having the function of the extracellular domain of SDC:
   (3A) a base sequence of SEQ ID NO: 6,
   (3B) a base sequence in which one to several amino acids are deleted, substituted or added in the base sequence of SEQ ID NO: 6,
   (3C) a base sequence which hybridizes under stringent conditions with a base sequence complementary to the base sequence of SEQ ID NO: 6, and
   (3D) a base sequence having 80% or more homology with the base sequence of SEQ ID NO: 6.
11. A recombinant cell in which a gene that encodes an extracellular domain of SDC is introduced into a mammalian cell that produces a heparin-like substance.
12. The recombinant cell according to 11, in which the mammalian cell that produces a heparin-like substance is a cell into which at least one of a gene that encodes NDST2 and a gene that encodes Hs3st1 is introduced.
13. The recombinant cell according to 11 or 12, in which the mammalian cell is a CHO cell.
14. A method for producing a cell that secretes and produces a heparin-like substance, the method comprising: introducing a recombinant expression vector comprising a gene that encodes an extracellular domain of SDC into a mammalian cell that produces a heparin-like substance.

### Effects of the Invention

According to the method for producing a heparin-like substance of the present invention, a heparin-like substance can be secreted and produced in a culture supernatant by introducing a gene that encodes an extracellular domain of syndecan into a mammalian cell that produces a heparin-like substance, and the heparin-like substance can be recovered from the culture supernatant. This solves the problem that a heparin-like substance as a product adheres to a cell membrane, and does not require a complicated purification process. Therefore, according to the production method of the present invention, a heparin-like substance can be efficiently produced without using an animal-derived tissue.

### Description of the Drawings

[FIG. 1] FIG. 1 is a diagram showing results of measuring an amount of sGAG in a culture supernatant of CHO/NH-SRGN cells and CHO/NH-SDC cells by an sGAG assay.
[FIG. 2] FIG. 2 is a diagram showing results of measuring the amount of sGAG in the culture supernatant of CHO/NH-SRGN cells and CHO/NH-SDC cells by HPLC.
[FIG. 3] FIGS. 3(A) and 3(B) are diagrams showing results of analyzing a sugar chain structure of heparin in the culture supernatant of CHO/NH-SRGN cells and CHO/NH-SDC cells using HPLC, in which FIG. 3(A) shows results of quantifying a concentration of a heparin-like sugar chain, and FIG. 3(B) shows results of quantifying a ratio of the heparin-like sugar chain in sulfation, respectively.
[FIG. 4] FIG. 4 is a graph showing results of measuring an amount of sGAG after serum-free acclimatization.
[FIG. 5] FIG. 5 is a graph showing results of calculating a production rate of sGAG after the serum-free acclimatization.
[FIG. 6] FIG. 6 shows results of measuring an expression level of a target protein by ELISA.
[FIG. 7] FIGS. 7(A) to 7(C) show results of measuring an expression level of an introduced gene by qRT-PCR, in which FIG. 7(A) shows an expression level of a NDST2 gene, FIG. 7(B) shows an expression level of an Hs3st1 gene, and FIG. 7(C) shows an expression level of an SDC gene.
[FIG. 8] FIG. 8 shows results of quantifying sGAG of a sample used for analysis of the sugar chain structure by HPLC.
[FIG. 9] FIG. 9 is a diagram showing chromatograms obtained from standard heparin-derived unsaturated disaccharides (HS-standards) and each sample by HPLC.
[FIG. 10] FIGS. 10(A) and 10(B) are diagrams showing results of analyzing the sugar chain structure of heparin in the culture supernatant of each cell using HPLC, in which FIG. 10(A) shows results of quantifying a concentration of a heparin-like sugar chain, and FIG. 10(B) shows results of quantifying a ratio of the heparin-like sugar chain in sulfation.
[FIG. 11] FIG. 11 shows a calibration curve used for evaluation of anticoagulation activity.

### Embodiments for Carrying Out the Invention

Hereinafter, the terms used in the present specification have meanings generally used in the field unless otherwise specified.

### <Method for producing heparin-like substance>

A method for producing a heparin-like substance according to the present invention comprises the following steps (1) to (3):
(1) preparing a mammalian cell that produces a heparin-like substance;
(2) preparing a recombinant cell in which a gene that encodes an extracellular domain of syndecan is introduced into the mammalian cell that produces a heparin-like substance and is prepared in step (1); and
(3) culturing the recombinant cell prepared in step (2) in a medium, and collecting a heparin-like substance from a resulting culture supernatant.

Hereinafter, each step will be described.

### Step (1): Preparing a mammalian cell that produces a heparin-like substance

Step (1) is a step of preparing a mammalian cell that produces a heparin-like substance by introducing a gene that encodes a protein necessary for heparin biosynthesis into a mammalian cell serving as a host.

In the present invention, the "heparin-like substance" refers to a mixture of heparin and HS.

Examples of the mammalian cell include Chinese hamster-derived ovary CHO cells [Journal of Experimental Medicine, 108, 945 (1958); Proc. Natl. Acad. Sci. USA, 60, 1275 (1968); Genetics, 55, 513 (1968); Chromosoma, 41, 129 (1973); Methods in Cell Science, 18, 115 (1996); Radiation Research, 148, 260 (1997); Proc. Natl. Acad. Sci. USA, 77, 4216 (1980); Proc. Natl. Acad. Sci., 60, 1275 (1968); Cell, 6, 121 (1975); Molecular Cell Genetics, Appedix I, II (pp. 883-900)]; CHO cells lacking a dihydrofolate reductase gene (CHO/DG44 cells) [Proc. Natl. Acad. Sci. USA, 77, 4216 (1980)], CHO-K1 (ATCCL-61), DUkXB11 (ATCC CCL-9096), Pro-5(ATCC CCL-1781), and CHO-S (Life Technologies, Cat# 11619) Pro-3, human umbilical vein endothelial cells (HUVEC), human umbilical artery endothelial cells (HUAEC), human lung microvascular vein endothelial cells (HLMVEC), human aortic endothelial cells (HAoEC), human coronary artery endothelial cells (HCAEC), human pulmonary artery endothelial cells (HPAEC), human embryonic kidney (HEK), rat myeloma cells YB2/3HL.P2.G11.16Ag.20 (also referred to as YB2/0"), monkey cells COS cells, mouse myeloma cells NSO, mouse myeloma cells SP2/0-Ag14, mouse myeloma cells BKK or HBT5637 (JP-A-S63-000299). Among them, CHO cells, CHO/DG44 cells, or CHO-K1 (ATCC CCL-61) are preferable, and CHO cells are more preferable, from the viewpoint of production efficiency.

Examples of the protein required for heparin biosynthesis include bifunctional heparan sulfate N-deacetylase/N-sulfate transferase (hereinafter abbreviated as NDST2), heparan sulfate ester glucosamine 3-sulfate transferase 1 (hereinafter abbreviated as Hs3st1), heparan sulfate 2-O-sulfate transferase, C5-epimerase, and heparan sulfate 6-O-sulfate transferase. Among these, NDST2 and Hs3st1 are preferable. These proteins may be variant proteins as long as these proteins have activity required for heparin biosynthesis. These proteins are preferably derived from mammals, and more preferably derived from mice or humans.

Examples of the protein necessary for heparin biosynthesis include a protein that cannot be expressed in wild-type mammalian cells. Specifically, for example, when mammalian cells are CHO cells, CHO cells that produce a heparin-like substance can be obtained by introducing at least one of genes that encode NDST2 and Hs3st1, which are necessary for heparin biosynthesis and are not expressed in CHO cells (Bail JY et al., Metab Eng 14: 81-90, 2012).

Examples of NDST2 include a protein comprising any one of the following amino acid sequences (1-a) to (1-c) and having a function of NDST2.
(1-a) Amino acid sequence of SEQ ID NO: 2 or amino acid sequence of NCBI accession NO: NP_003626.1
(1-b) Amino acid sequence in which one to several amino acids are deleted, substituted or added in amino acid sequence of SEQ ID NO: 2 or amino acid sequence of NCBI accession NO: NP_003626.1
(1-c) Amino acid sequence having 60% or more, preferably 80% or more, more preferably 90% or more, and most preferably 95% or more homology, with amino acid sequence of SEQ ID NO: 2 or amino acid sequence of NCBI accession NO: NP_003626.1

Examples of Hs3st1 include a protein comprising any one of the following amino acid sequences (2-a) to (2-c) and having a function of Hs3st1.
(2-a) Amino acid sequence of SEQ ID NO: 3 or amino acid sequence of NCBI accession NO: NP_034604.1
(2-b) Amino acid sequence in which one to several amino acids are deleted, substituted or added in amino acid sequence of SEQ ID NO: 3 or amino acid sequence of NCBI accession NO: NP_034604.1
(2-c) Amino acid sequence having 60% or more, preferably 80% or more, more preferably 90% or more, and most preferably 95% or more homology, with amino acid sequence of SEQ ID NO: 3 or amino acid sequence of NCBI accession NO: NP_034604.1

A polypeptide comprising an amino acid sequence in which one to several amino acids have been deleted, substituted or added in a target amino acid sequence can be obtained by introducing a site-specific mutation into DNA that encodes a polypeptide comprising the amino acid sequences of SEQ ID NO: 1 to 3, for example, by using a site-specific mutagenesis method [Molecular Cloning, A Laboratory Manual, Second Edition, Cold Spring Harbor Laboratory Press (1989), Current Protocols in Molecular Biology, John Wiley & Sons (1987-1997), Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985)].

A range of "one to several" in "one to several amino acids are deleted, substituted or added" in the amino acid sequence is not particularly limited, but for example, when the number of amino acids in the amino acid sequence is 100 as one unit, "one to several" means 1, 2, 3, 4, 5, 6, 7, 8, 9, 10, 11, 12, 13, 14, 15, 16, 17, 18, 19, or 20, preferably about 1, 2, 3, 4, 5, 6, 7, 8, 9, or 10, and more preferably about 1, 2, 3, 4, or 5, per unit.

The term "amino acids are deleted" means loss or disappearance of an amino acid residue in the sequence, the term "amino acids are substituted" means that an amino acid residue in the sequence is replaced with another amino acid residue, and the term "amino acids are added" means that a new amino acid residue is added so as to be inserted into the sequence.

As a specific aspect of "one to several amino acids are deleted, substituted or added", there is an aspect in which one to several amino acids are replaced with other chemically similar amino acids. Examples thereof include a case where a certain hydrophobic amino acid is substituted with another hydrophobic amino acid and a case where a certain polar amino acid is substituted with another polar amino acid having the same charge. Such chemically similar amino acids are known in the art for each amino acid.

Specific examples of a nonpolar (hydrophobic) amino acid include alanine, valine, glycine, isoleucine, leucine, proline, tryptophan, phenylalanine, and methionine. Examples of a polar (neutral) amino acid include serine, threonine, tyrosine, glutamine, asparagine, and cysteine. Examples of a basic amino acid having a positive charge include arginine, histidine, and lysine. Examples of an acidic amino acid having a negative charge include aspartic acid and glutamic acid.

Examples of the amino acid sequence having deletion, substitution, addition, or the like of one to several amino acids in the amino acid sequence of the target protein include an amino acid sequence having a certain sequence identity or more with the amino acid sequence of the target protein, and examples thereof include an amino acid sequence having 60% or more, preferably 65% or more, preferably 70% or more, preferably 75% or more, preferably 80% or more, preferably 85% or more, more preferably 90% or more, and still more preferably 95% or more sequence identity with the amino acid sequence of the target protein.

Examples of the gene that encodes NDST2 include a gene comprising a base sequence that is any one of the following base sequences (1-A) to (1-D) and encodes an amino acid sequence of a protein having a function of NDST2.
(1-A) Base sequence of SEQ ID NO: 4 or base sequence of NCBI accession NO: NM_003635.3
(1-B) Base sequence in which one to several bases are deleted, substituted or added in base sequence of SEQ ID NO: 4 or base sequence of NCBI accession NO: NM_003635.3
(1-C) Base sequence which hybridizes under stringent conditions with base sequence complementary to base sequence of SEQ ID NO: 4 or base sequence of NCBI accession NO: NM_003635.3
(1-D) Base sequence having at least 60% or more, preferably 80% or more, and more preferably 95% or more homology, with base sequence of SEQ ID NO: 4 or base sequence of NCBI accession NO: NM_003635.3

Examples of the gene that encodes Hs3st1 include a gene comprising a base sequence that is any one of the following base sequences (2-A) to (2-D) and encodes an amino acid sequence of a protein having a function of Hs3st1.
(2-A) Base sequence of SEQ ID NO: 5 or base sequence of NCBI accession NO: NM_010474.2
(2-B) Base sequence in which one to several bases are deleted, substituted or added in base sequence of SEQ ID NO: 5 or base sequence of NCBI accession NO: NM_010474.2
(2-C) Base sequence which hybridizes under stringent conditions with base sequence complementary to base sequence of SEQ ID NO: 5 or base sequence of NCBI accession NO: NM_010474.2
(2-D) Base sequence having at least 60% or more, preferably 80% or more, and more preferably 95% or more homology, with base sequence of SEQ ID NO: 5 or base sequence of NCBI accession NO: NM_010474.2

The base sequence which hybridizes under stringent conditions refers to a base sequence of hybridizable DNA obtained by a colony hybridization method, a plaque hybridization method, a Southern blot hybridization method, a DNA microarray method, or the like, using DNA comprising a target base sequence as a probe.

Specific examples thereof include a base sequence of DNA that can be identified by performing a hybridization method [Molecular Cloning, A Laboratory Manual, Second Edition, Cold Spring Harbor Laboratory Press (1989), Current Protocols in Molecular Biology, John Wiley & Sons (1987-1997), DNA Cloning 1: Core Techniques, A Practical Approach, Second Edition, Oxford University, (1995)] at 65°C in the presence of 0.7 mol/L to 1.0 mol/L of sodium chloride using hybridized colony or plaque derived DNA, or a filter or slide glass on which a PCR product or oligo DNA having the sequence is immobilized, and then washing the filter or slide glass at 65°C using an SSC solution (a composition of the SSC solution at a concentration of 1 time is composed of 150 mmol/L sodium chloride and 15 mmol/L sodium citrate) at a concentration of 0.1 to 2 times.

Examples of DNA comprising a base sequence which hybridizes under stringent conditions include DNA having a certain level or more of sequence identity with a base sequence of DNA having a base sequence of a gene to be used as a probe. For example, DNA having at least 60% or more, preferably 80% or more, and more preferably 95% or more homology, with the target base sequence is exemplified. For example, when the number of bases in the base sequence is 100 as one unit, DNA is exemplified comprising a base sequence having deletion, substitution, addition, or the like of one to several bases, preferably 1 to 40 bases, preferably 1 to 35 bases, preferably 1 to 30 bases, preferably 1 to 25 bases, preferably 1 to 20 bases, more preferably 1 to 15 bases, still more preferably 1, 2, 3, 4, 5, 6, 7, 8, 9, or 10 bases, and yet still more preferably 1, 2, 3, 4, or 5 bases, per unit in the base sequence of the target gene.

The term "deletion of a base" means that the base in the sequence is lost or disappeared, the term "substitution of a base" means that the base in the sequence is replaced with another base, and the term "addition of a base" means that a new base is added so as to be inserted.

The base sequence of a gene that encodes a protein of a eukaryote often shows polymorphism of a gene. The gene used in the present invention also contains a gene having a small mutation in the base sequence due to such a polymorphism.

A numerical value of homology in the present invention may be a numerical value calculated using a homology search program known to those skilled in the art, unless otherwise specified, and examples of the numerical value of homology in the present invention include a numerical value calculated using default parameters in BLAST [J. Mol. Biol., 215, 403 (1990)] for the base sequence, and a numerical value calculated using default parameters in BLAST 2 [Nucleic Acids Res.,25, 3389 (1997),Genome Res., 7, 649 (1997), https://blast.ncbi.nlm.nih.gov/Blast.cgi?PAGE_TYPE=BlastSearch&BLAST_SPEC=blast 2seq&LINK_LOC=align2seq] for the amino acid sequence.

The default parameters are: G(Cost to open gap) is 5 for a base sequence and 11 for an amino acid sequence, -E(Cost to extend gap) is 2 for a base sequence and 1 for an amino acid sequence, -q(Penalty for nucleotide mismatch) is -3, -r (reward for nucleotide match) is 1, -e (expect value) is 10, -W (wordsize) is 11 residues for a base sequence and 3 residues for an amino acid sequence, -y[Dropoff(X) for blast extensions in bits] is 20 for blastn, 7 for non-blastn programs, -X(X dropoff value for gapped alignment in bits) is 15 and -Z(final X dropoff value for gapped alignment in bits ) is 50 for blastn and 25 for non-blastn programs (https://blast.ncbi.nlm.nih.gov/Blast.cgi?PAGE_TYPE=BlastSearch).

The mammalian cell that produces a heparin-like substance can be obtained by introducing a recombinant vector comprising cDNA that encodes a full length or partial length of a protein required for heparin biosynthesis into a mammalian cell serving as a host cell.

As the recombinant vector, any vector can be used as long as the vector can be autonomously replicated or incorporated into a chromosome in a host cell to be used and contains an appropriate promoter at a position where DNA that encodes a polypeptide can be transcribed.

Although a transcription termination sequence is not necessarily required for the recombinant vector, it is preferable to arrange the transcription termination sequence immediately below a structural gene. Furthermore, the recombinant vector may contain a gene that controls a promoter.

As the recombinant vector, it is preferable to use a plasmid in which a Kozak sequence, which is a ribosomal binding sequence, is appropriately arranged around a start codon.

In a base sequence of DNA, the base can be substituted so as to be an optimal codon for expression in the host, thereby improving a production rate of the target NDST2 and Hs3st1.

As the recombinant vector, any vector can be used as long as the vector can exhibit functions in animal cells, and examples thereof include pcDNA I, pcDM8 (Funakoshi Corp.), pAGE107 [JP-A-03-22979; Cytotechnology, 3, 133 (1990)], pAS3-3 (JP-A-02-227075), pcDM8 [Nature, 329, 840 (1987)), pcDNAI/Amp (Invitrogen Corp.], pcDNA3.1 (Invitrogen Corp.), pREP4 (Invitrogen Corp.), pAGE103 [J. Biochemistry, 101, 1307 (1987)], pAGE210, pME18SFL3, pKANTEX93 (WO 97/10354), N5KG1val (specification of US Patent No. 6001358), INPEP4 (Biogen-IDEC Corp.), and a transposon vector (WO 2010/143698).

As the promoter, any promoter can be used as long as the promoter can exhibit functions in animal cells, and examples thereof include a promoter of an immediate early (IE) gene of cytomegalovirus (CMV), a SV40 early promoter, a retrovirus promoter, a metallothionein promoter, a heat shock promoter, an SRα promoter, and a promoter or enhancer of Moloney murine leukemia virus. An enhancer of the IE gene of human CMV may be used together with a promoter.

The recombinant vector may comprise a selection marker. The "selection marker" is a gene that enables selection of cells comprising the gene. The term "positive selection" refers to a process by which a positive selection occurs and a cell comprising a selection marker is selected. An example of the selection marker with positive drug resistance is that a cell comprising the marker survives in a drug-containing culture medium, and a cell without the marker is killed. Examples of the selection marker include a drug resistance gene such as neo giving G418 resistance; hygr giving hygromycin resistance; and puro giving puromycin resistance. Other positive selection marker genes include a gene that enables identification or screening of cells comprising the marker. Examples of these genes include a fluorescent protein (GFP and GFP-like chromophore, luciferase) gene, a lacZ gene, an alkaline phosphatase gene, and a surface marker such as CD8. The term "negative selection" refers to a process by which a cell comprising a negative selection marker is killed by being exposed to an appropriate negative selection drug. For example, a cell comprising a simple herpesvirus kinase (HSV-tk) gene [Wigler et al, Cell 11: 223 (1977)] is sensitive to a drug gamma cyclobilla (GANC). Similarly, a gpt gene makes cells sensitive to 6-thioxanthine.

As a method for introducing a recombinant vector into a host cell, any method for introducing DNA into an animal cell can be used. Examples thereof include an electroporation method [Cytotechnology, 3, 133 (1990)], a calcium phosphate method (JP-A-02-227075), and a lipofection method [Proc. Natl. Acad. Sci. USA, 84, 7413 (1987)].

Step (2): Preparing a recombinant cell in which a gene that encodes an extracellular domain of syndecan is introduced into the mammalian cell that produces a heparin-like substance and is prepared in step (1)

Step (2) is a step of preparing a recombinant cell by introducing a recombinant vector comprising a gene that encodes an extracellular domain of syndecan into the mammalian cell that produces a heparin-like substance and prepared in step (1).

A structure of syndecan comprises an extracellular domain, a transmembrane domain, and a cytoplasmic domain. Among them, the extracellular domain of syndecan comprises a glycosaminoglycan binding site. It is preferable that syndecan has at least a glycosaminoglycan binding site as an extracellular domain. The extracellular domain of syndecan is preferably derived from a mammal, and more preferably derived from a mouse or a human.

Examples of the extracellular domain of syndecan include a protein comprising any one of the following amino acid sequences (3-a) to (3-c) and having a function of the extracellular domain of syndecan.
(3-a) Amino acid sequence of SEQ ID NO: 1 or amino acid sequence of NCBI accession NO: NP_001006947.1
(3-b) Amino acid sequence in which one to several amino acids are deleted, substituted or added in amino acid sequence of SEQ ID NO: 1
(3-c) An amino acid sequence having 60% or more, preferably 80% or more, more preferably 90% or more, and most preferably 95% or more homology, with amino acid sequence of SEQ ID NO: 1

The amino acid sequence of SEQ ID NO: 1 is an amino acid sequence at positions 1 to 229 from the N-terminal of the amino acid sequence of NCBI accession number NP_001006947.1 (total length 310 amino acids).

Examples of the gene that encodes the extracellular domain of syndecan include a gene comprising a base sequence that is any one of the following base sequences (3-A) to (3-D) and encodes an amino acid sequence of a protein having the function of an extracellular domain of syndecan.
(3-A) Base sequence of SEQ ID NO: 6 or base sequence of NCBI accession NO: NM_001006946.1
(3-B) Base sequence in which one to several bases are deleted, substituted or added in base sequence of SEQ ID NO: 6 or base sequence of NCBI accession NO: NM_001006946.1
(3-C) Base sequence which hybridizes under stringent conditions with base sequence complementary to base sequence of SEQ ID NO: 6 or base sequence of NCBI accession NO: NM_001006946.1
(3-D) Base sequence having at least 60% or more, preferably 80% or more, and more preferably 95% or more homology, with base sequence of SEQ ID NO: 6 or base sequence of NCBI accession NO: NM_001006946.1

In step (2), a recombinant cell that can secrete a heparin-like substance into a culture supernatant is obtained by introducing the recombinant vector comprising the gene that encodes the extracellular domain of syndecan, that is, cDNA that encodes a whole length or partial length of the extracellular domain of syndecan into the mammalian cell that produces a heparin-like substance and is prepared in step (1).

A method for introducing a recombinant vector and a recombinant vector into a host cell is the same as in step (1).

### (3) Culturing the recombinant cell prepared in step (2) in a medium, and collecting a heparin-like substance from a resulting culture supernatant

Step (3) is a step of culturing the recombinant cell prepared in step (2) in a medium to secrete and produce a heparin-like substance in the medium by the recombinant cell, and recovering the heparin-like substance from the resulting culture supernatant.

In step (3), the recombinant cell is preferably cultured under conditions for promoting the production of the heparin-like substance. Specifically, for example, it is preferable to culture the recombinant cell in a medium that enables production of the heparin-like substance in the recombinant cell and promotes secretion of the heparin-like substance from the recombinant cell into the culture supernatant.

The medium preferably contains carbon, nitrogen, oxygen, and other nutrients, a growth factor, a buffer, a cofactor, and other optional substances sufficient to maintain at least viability of cells and enable expression of heparin-like substances. In the embodiment in which the gene that encodes a heparin-like substance is under control of an inducible promoter or contains an inducible promoter, the medium may further contain an inducer.

Examples of the medium include RPMI or DMEM to which 10% fetal bovine serum (FCS) is added, and a tissue culture medium to which a factor such as an antimicrobial agent, a growth factor, and other cytokines is added (for example, Cell Biology (Third Edition) A Laboratory Handbook, vol. 1, 2006, Elsevier Inc.). Specific examples thereof include a medium preparation known to those skilled in the art, such as RPMI, IMDM, DMEM, DMEM/F12, and serum-free or low-serum EMEM. These media may contain an additional nutritional supplement food such as antibiotics, lipids, transferrin, insulin, and amino acids, and as necessary, a cofactor.

From the viewpoint of promoting the production of the heparin-like substance, the medium preferably contains at least one selected from glucose, sulfates, and phosphoric acid. A concentration of the glucose in the medium is usually preferably 5 mM to 75 mM, more preferably 10 mM to 60 mM, and still more preferably 15 mM to 35 mM. A concentration of the sulfate in the medium is usually preferably 0.5 mM to 50 mM, more preferably 10 mM to 50 mM, and still more preferably 30 mM to 50 mM. A concentration of the phosphate in the medium is usually preferably 0.5 mM to 50 mM, more preferably 1 mM to 50 mM, and still more preferably 10 mM to 50 mM.

The heparin-like substance can be produced by producing and accumulating the heparin-like substance in the culture supernatant and collecting the heparin-like substance from the culture supernatant. A method of culturing a recombinant cell in a medium can be performed according to a usual method. Culture is usually performed for 1 day to 7 days under conditions such as pH 6 to 8, 30°C to 40°C, and 5% CO₂.

The secretion and production of the heparin-like substance from the recombinant cell can be confirmed by adding an enzyme solution containing heparin-lyase I, II, or III to a culture supernatant, subjecting the culture supernatant to an enzyme treatment, and then performing quantitative determination by HPLC for unsaturated disaccharide analysis, as will be described later in Examples. Specifically, it is assumed that the heparin-like substance is secreted and produced from the recombinant cell into the culture supernatant when an amount of at least one selected from 2SNS6 in which an amino group of glucosamine and three sulfate groups are bonded at 6-position, 2SNS in which a sulfate group is bonded to 2-position glucuronic acid and a glucosamine amino group, NS6S in which an amino group of glucosamine and two sulfates are bonded at 6-position, and NS in which a sulfate group is bonded to glucosamine N is increased in the culture supernatant of the recombinant cell as compared with the culture supernatant of a control (wild-type) cell. The secretion production of the heparin-like substance from the recombinant cell can also be confirmed by measuring an amount of sGAG (sulfated GAG) in the culture supernatant, as will be described later in Examples.

In step (3), proteoglycan, which is a glycoprotein comprising a core protein bound to a heparin-like substance, which is GAG, is secreted from the recombinant cell into the culture supernatant. Isolation of proteins from the culture supernatant is performed by a common method known in the art. For example, a tag that facilitates isolation of the heparin-like substance, such as an affinity tag, may be used. Examples of the tag include polyhistidine (His6 tag), a nickel matrix, a chitin binding protein (CBP), a maltose binding protein (MBP), glutathione-S-transferase (GST), a FLAG tag, and an epitope tag.

Isolation of the heparin-like substance from the core protein is performed by a method known in the art. Examples thereof include an enzyme digestion method using heparinase, and a treatment with sodium hydroxide or alkaline boron hydride.

The heparin-like substance to be isolated preferably contains 50% or more, more preferably 60% or more, still more preferably 70% or more, particularly preferably 80% or more of a sulfated disaccharide.

The heparin-like substance to be isolated includes, for example, the following (1) to (20).
(1) 10% to 50% of trisulfated disaccharide, 30% to 50% of disulfated disaccharide, and 10% to 30% of monosulfated disaccharide.
(2) 10% to 40% of trisulfated disaccharide, 35% to 45% of disulfated disaccharide, and 10% to 25% of monosulfated disaccharide.
(3) 15% to 40% of trisulfated disaccharide, 35% to 45% of disulfated disaccharide, and 10% to 25% of monosulfated disaccharide.
(4) 5% to 25% of trisulfated disaccharide, 30% to 50% of disulfated disaccharide, and 10% to 20% of monosulfated disaccharide.
(5) 1% to 30% of trisulfated disaccharide, 25% to 55% of disulfated disaccharide, and 5% to 40% of monosulfated disaccharide.
(6) 15% to 40% of trisulfated disaccharide.
(7) 35% to 45% of disulfated disaccharide.
(8) 10% to 25% of monosulfated disaccharide.
(9) 10% to 50% of trisulfated disaccharide.
(10) 30% to 50% of disulfated disaccharide.
(11) 10% to 30% of monosulfated disaccharide.
(12) 15% to 30% of trisulfated disaccharide.
(13) 40% to 45% of disulfated disaccharide.
(14) 10% to 15% of monosulfated disaccharide.
(15) 5% to 25% of trisulfated disaccharide.
(16) 1% to 30% of trisulfated disaccharide.
(17) 30% to 50% of disulfated disaccharide.
(18) 25% to 55% of disulfated disaccharide.
(19) 10% to 20% of monosulfated disaccharide.
(20) 5% to 40% of monosulfated disaccharide.

The heparin-like substance may contain a repeating structure of a disaccharide unit having various lengths. The heparin-like substance preferably contains any of UA-GlcNAc(6S), UA(2S)-GlcNAc, UA-(2S)-GlcNAc(6S), UA-GlcNS, UA-GlcNS(6S), UA(2S)-GlcNS, and UA(2S)-GlcNS(6S), and these may be present in any order in the heparin-like substance.

In the above, UA is a uronic acid residue (that is, glucuronic acid or iduronic acid), Ac is acetyl, GlcNAc is N-acetyl glucosamine, GlcNS is glucosamine-N-sulfate, 2S is 2-O-sulfonate, and 6S is 6-O-sulfate.

### <Recombinant cell>

An aspect of the present invention is a recombinant cell in which a gene that encodes an extracellular domain of SDC is introduced into the mammalian cell that produces a heparin-like substance. The recombinant cell can be prepared by preparing a mammalian cell that produces a heparin-like substance and introducing a gene that encodes an extracellular domain of syndecan into the mammalian cell in the same manner as described above in step (1) and step (2) of <Method for producing heparin-like substance>.

### <Method for producing cell that secretes and produces heparin-like substance>

An aspect of the present invention is a method for producing a cell that secretes and produces a heparin-like substance. The method comprises: introducing a recombinant expression vector comprising a gene that encodes an extracellular domain of SDC into the mammalian cell that produces a heparin-like substance. The production method can be carried out by preparing a mammalian cell that produces a heparin-like substance and introducing a gene that encodes an extracellular domain of syndecan into the mammalian cell in the same manner as described above in steps (1) and (2) of <Method for producing heparin-like substance>.

### <Pharmaceutical composition>

An aspect of the present invention is a pharmaceutical composition comprising a heparin-like substance or a fragment thereof produced by the method described in the present specification. In the present embodiment, the heparin-like substance or the fragment thereof may bind to the core protein. The pharmaceutical composition may contain other therapeutic agents. The pharmaceutical composition can be formulated by using, for example, a common vehicle or diluent, and a pharmaceutical additive (for example, an excipient, a binder, or a preservative) of a type suitable for a desired administration method.

Examples of a form of the pharmaceutical composition include an aqueous agent for sterile injection. The aqueous agent for sterile injection can be formulated according to a known technique by using an appropriate dispersant or wetting agent and suspending agent. The aqueous agent for sterile injection may be, for example, a nontoxic parenterally acceptable diluent such as a solution in 1,3-butanediol, or a solution or suspension for sterile injection in a solvent. The vehicle and the solvent that can be used are water, a Ringer's solution, and an isotonic saline solution.

A dosage form of the pharmaceutical composition is not particularly limited, and various dosage forms can be used. Examples of the dosage form for administering pharmaceutical compositions includes the composition being administered in a form of a tablet, a capsule, a sachet, a troche, a pill, a powder, a granule, an elixer, a tincture, a solution, a suspending agent, an elixer, a syrup, an ointment, a cream, or the like, intravenous administration or injection, a paste, an emulsion or a solution. In addition, examples thereof include transdermal administration by a patch mechanism or an ointment. Any of these may be modified into a sustained release preparation and/or a continuous release preparation.

Examples of a pharmaceutically acceptable carrier include, but are not limited to, a vehicle, an adjuvant, a surfactant, a suspending agent, an emulsifier, an inert filler, a diluent, an excipient, a wetting agent, a binder, a lubricant, a buffer, a disintegrating agent, and a carrier. Typically, the pharmaceutically acceptable carrier is chemically inactive to an active compound, and does not have harmful side effects or toxicity under use conditions. Properties of the pharmaceutically acceptable carrier may vary depending on other features of the specific dosage form and the composition to be used.

### <Treatment method and preventing method>

An aspect of the present invention includes a method for treating or preventing blood coagulation or a state associated with or caused by blood coagulation in a subject requiring a heparin-like substance or a fragment thereof. The method comprises administering the heparin-like substance or the fragment thereof produced by the method described in the present specification. In the present embodiment, the heparin-like substance or the fragment thereof may bind to the core protein. Examples of the state associated with or caused by blood coagulation include acute coronary syndrome, atrial fibrillation, deep venous thrombosis, and pulmonary embolization.

The subject refers to a mammal. Examples of the mammal include a human, a primate, a livestock animal (for example, sheep, bovine, horse, donkey, pig), a companion animal (for example, dog, cat), a laboratory test animal (for example, mouse, rabbit, rat, guinea pig, hamster), and a capture wild animal (for example, rice, sika). The mammal is typically a human or a primate, and more typically a human.

It is preferable that a dose and dosing time is a dose and time at which a therapeutic profit is provided in treatment, prevention, or management of blood coagulation or a state associated with blood coagulation. A specific effective dose and dosing time may vary depending on a factor such as a state, a medical history, a physique, a body weight, and an age of the subject.

### Example

Examples will be described below, but the present invention is not limited to the following Examples.

### 1. Evaluation of heparin-producing ability in CHO/NH-SRGN cells and CHO/NH-SDC cells

### <Preparation of CHO/NH-SRGN cells and CHO/NH-SDC cells>

Based on Bail JY et al., Metab Eng14:81-90, 2012, DNA that encodes NDST2 (base sequence represented by SEQ ID NO: 4) and DNA that encodes Hs3st1 (base sequence represented by SEQ ID NO: 5) were co-introduced to obtain a CHO/NH cell clone #3 (CHO-NH #3 cell).

A vector PB513B-1_SRGN-IRES-EGFP used to prepare CHO/NH-SRGN cells was based on PiggyBac Transposon Vector PB513B-1, and was a vector having an EF1α promoter of a Chinese hamster, an IRES element, and a mycHis tag sequence at C-terminus. In the vector PB513B-1_SRGN-IRES-EGFP, DNA that encodes hSRGN (base sequence represented by SEQ ID NO: 7) was introduced under the control of the EF1α promoter so as to be co-expressed with an EGFP gene and a puromycin resistance gene, which are screening markers.

CHO/NH-SRGN cells were prepared by introducing DNA that encodes human sericin glycine (SRGN) (base sequence represented by SEQ ID NO: 7) into CHO/NH #3 cells using a vector PB513B-1_SRGN-IRS-EGFP, and CHO/NH-SDC cells were prepared by introducing DNA of an extracellular domain of human syndecan (SDC) (base sequence represented by SEQ ID NO: 6) into CHO/NH #3 cells using a vector PB513B-1SDC-IRS-EGFP.

A vector PB513B-1SDC-IRES-EGFP used to prepare CHO/NH-SDC cells was based on PiggyBac Transposon Vector PB513B-1, and was a vector having an EF1α promoter of a Chinese hamster, an IRES element, and a mycHis tag sequence at C-terminus. In the vector PB513B-1_SDC-IRES-EGFP, DNA that encodes SRGN (base sequence represented by SEQ ID NO: 7) was introduced under the control of the EF1α promoter so as to be co-expressed with an EGFP gene and a puromycin resistance gene, which are screening markers.

The culture supernatant of each cell prepared as described above was analyzed to evaluate heparin-producing ability.

### <Measurement of amount of sGAG in culture supernatant of CHO/NH-SRGN cells and CHO/NH-SDC cells>

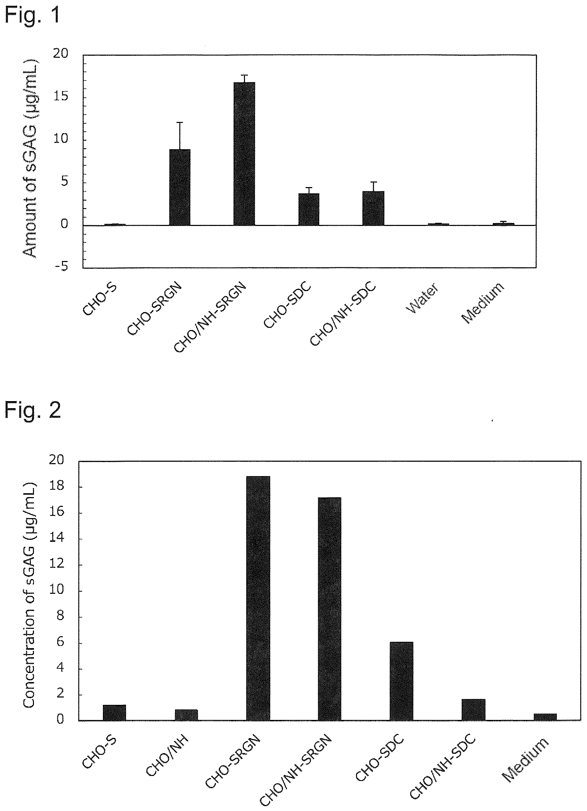

Each cell was cultured in a good proliferation state, and then seeded in a 6-well plate at 0.6 × 10⁶ cells/well, and a culture supernatant collected after 3 days without medium change was used as a sample to measure an amount of sGAG by an sGAG assay. The sGAG assay is an assay that colorimetrically quantifies a total amount of sGAG in a solution using a dye (1,9-dimethylmethylene blue) that specifically binds to a sulfated sugar chain. The results are shown in FIG. 1.

### <Analysis results of sugar chain structure by HPLC>

Since the production of heparin in the culture supernatant was suggested by the sGAG assay, the sugar chain structure of the same sample was analyzed by HPLC. The sGAG quantification results of the samples used for the analysis are shown in FIG. 2. Since a concentration of a CHO/NH-SDC cell sample was too low to confirm a presence of sufficient sugar chains for analysis, a spin column enrichment operation was performed.

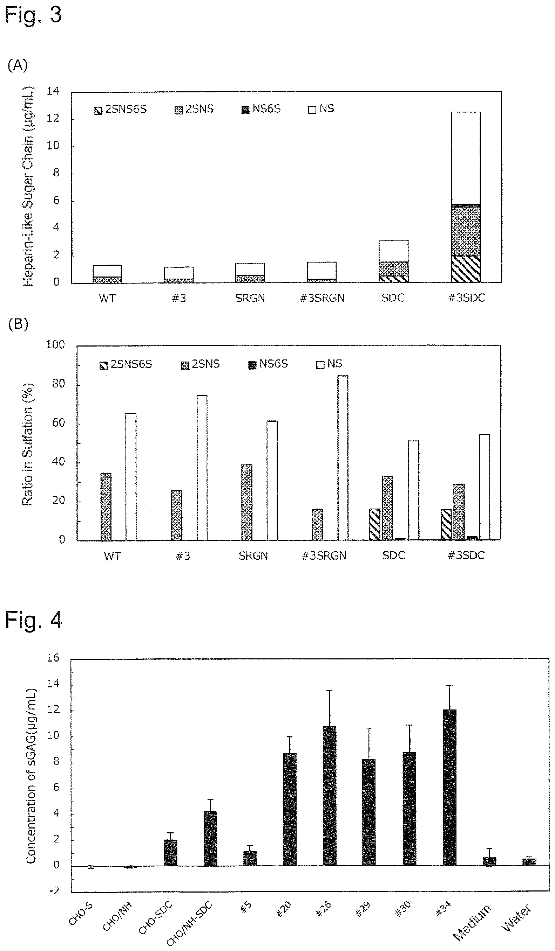

To confirm the production of heparin, first, 20 µL of a sample solution was directly taken, 10 µL of a buffer and 10 µL of an enzyme solution containing 10 mU of heparin lyase I, II or III were added thereto, the mixture was enzymatically treated at 37°C for 3 hours, after completion of the reaction, the enzyme was deactivated at 100°C for 5 minutes, 20 µL of water was added after freeze-drying, and the amount was determined by HPLC for unsaturated disaccharide analysis. The results are shown in FIGS. 3A and 3B.

In FIGS. 3(A) and 3(B), WT represents a wild-type CHO cell, #3 represents a CHO-NH #3 cell, SRGN represents a CHO-SRGN cell prepared by introducing DNA that encodes SRGN (base sequence represented by SEQ ID NO: 7) into a CHO cell using a vector PB513B-1_SRGN-IRES-EGFP, #3 SRGN represents a CHO/NH-SRGN cell, and SDC represents a CHO-SDC cell obtained by introducing DNA of an extracellular domain of SDC (base sequence represented by SEQ ID NO: 6) into a CHO cell using a vector PB513B-1_SDC-IRES-EGFP.

As shown in FIGS. 3(A) and 3(B), in the culture supernatant of the SRGN-introduced cells (SRGN, #3 SRGN), no increase in the detection of heparin-like substances was detected as compared with WT or #3. On the other hand, in the culture supernatant of the SDC-introduced cells (SDC, #3 SDC), the production of heparin-like substances was increased as compared with WT or #3. From the above results, the CHO/NH-SDC cells were considered to be cells more suitable for producing heparin-like substances, and cloning and analysis of each clone were performed.

### 2. Measurement results of amount of GAG after serum-free acclimatization

Since a sugar chain structure characteristic of heparin was observed, cloning of CHO/NH-SDC cells and analysis of each clone were carried out.

### <Cloning and serum-free acclimatization>

CHO/NH-SDC cells were cultured using a serum-containing medium to be in an adhered state, and cloned in a collagen-coated 96-well plate by a limiting dilution method. At this time, for the culture from one cell, the culture was performed without adding a drug in consideration of the toxicity to the cell. Cells were scaled up with a collagen-coated 24- and 6-well plate, a plurality of clones were cultured, and 36 clones were established. Thereafter, each cell was seeded in a normal 6-well plate at 0.6 × 10⁶ cells/well, and the amount of sGAG in the culture supernatant was measured using, as a sample, a culture supernatant collected three days without medium exchange, and an upper 6 clones (#5, #20, #26, #29, #30, and #34) were subjected to serum-free acclimatization.

### <Measurement of amount of sGAG, production rate of sGAG, and amount of SDC after serum-free acclimatization>

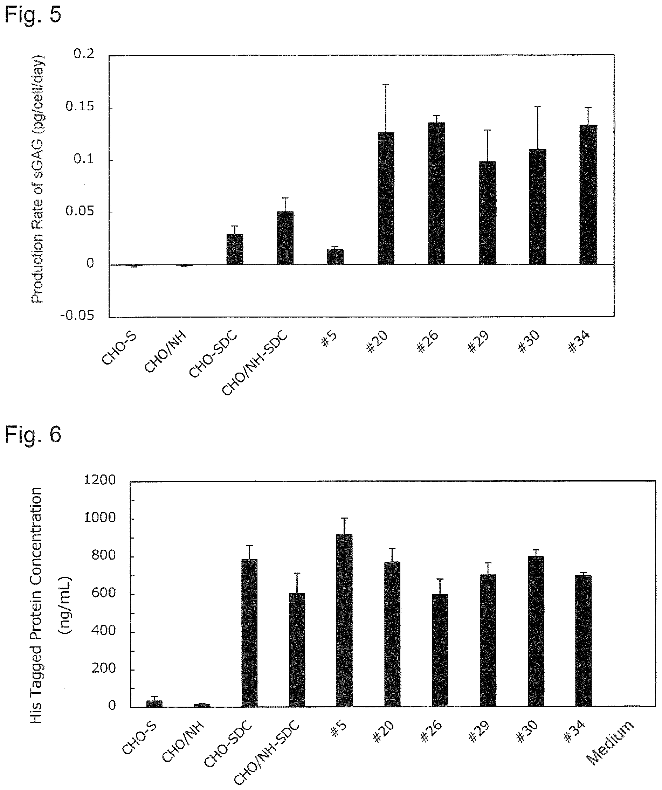

After the serum-free acclimatization, each cell was seeded on a normal 6-well plate at 0.6 × 10⁶ cells/well, and the amount of sGAG in the culture supernatant was measured using, as a sample, the culture supernatant collected 3 days without medium exchange. The results are shown in FIG. 4. The results of calculating the production rate of sGAG are shown in FIG. 5, and the results of quantifying the amount of SDC in the culture supernatant by ELISA are shown in FIG. 6.

As shown in FIG. 6, the evaluated 6 clones had the secretory production ability of SDC equal to or higher than that of CHO/NH-SDC cells, and #5 was 1.5 times or more that of CHO/NH-SDC cells, showing the highest expression level.

### <Analysis of expression level of introduced gene by qRT-PCR>

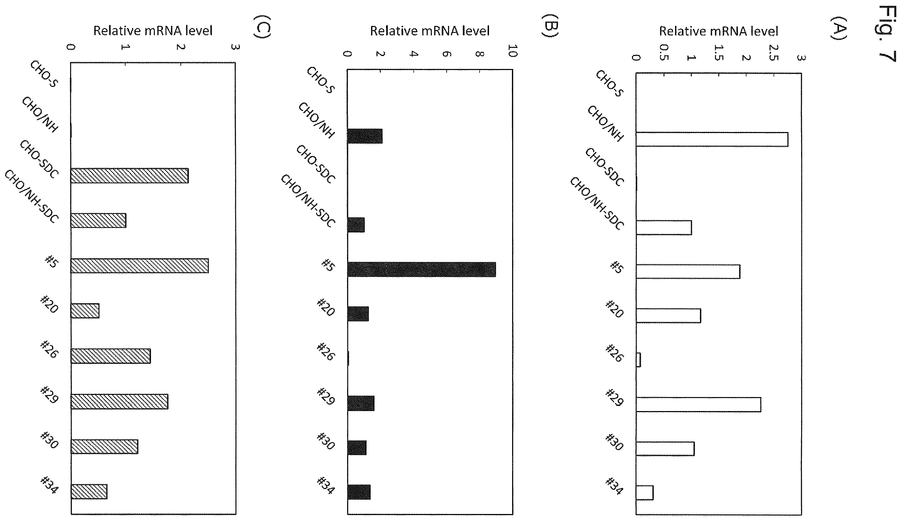

Each cell was seeded in a 6-well plate at 0.6 × 10⁶ cells/well, RNA was extracted and cDNA was prepared from cells cultured for 3 days without medium exchange, and expression levels of introduced genes (NDST2, Hs3st1 and SDC) were quantified by qRT-PCR. FIG. 7(A) shows an expression level of a NDST2 gene, FIG. 7(B) shows an expression level of an Hs3st1 gene, and FIG. 7(C) shows an expression levels of an SDC gene.

As shown in FIGS. 7(A) to 7(C), a high expression level of each gene was confirmed in #5, whereas no expression was observed in #26. #26 also showed low anticoagulant activity compared to other clones, suggesting that ATIII recognition-specific sequences are formed by NDST2 and Hs3st1.

### 3. Analysis of sugar chain structure by HPLC

Sugar chain structures of heparin-like substances produced by various CHO cells were analyzed by HPLC.

### <Analysis conditions>

As a pretreatment of a sample, 400 µL of culture supernatant of various CHO cells was centrifuged at 10000 rpm for 10 minutes, and 400 µL of two-stage distilled water (DDW) was added to an upper layer, followed by freeze-drying. Thereafter, 20 µL of the sample was separated from 40 µL of sample, subjected to Heparin lyase I, II and III treatments, 80 µL of DDW was added thereto, and 20 µL of the resultant was subjected to HPLC.

The treatment with a specific degrading enzyme was as follows. 20 µL of an acetate buffer [0.1 MCH₃COONa, 10mM (CH3COO)₂Ca (pH7.0)], 5 µL of 0.5 mU/µL Heparinase, 5 µL of 0.5 mU/µL Heparinase I, and 5 µL of 0.5 mU/µL Heparinase II were simultaneously added, and the mixture was incubated at 37°C for 16 hours to decompose heparin-like sugar chains into unsaturated disaccharides. The mixture was heated in a boiling water bath for 3 minutes to deactivate the enzyme, lyophilized, dissolved in water, and subjected to an unsaturated disaccharide analysis by HPLC.

### <Disaccharide composition analysis of HS and CS>

A disaccharide composition analysis of HS and CS was performed using a device and analysis software shown below.
Pump of eluent: LIQUID CHROMATOGRAPHY LC-10Ai manufactured by Shimadzu Corporation
Detector: Intelligent Fluorescence Detector FP-920S manufactured by JASCO
Pump for post reaction: MINICHEMI PUMP NP-FX(II)-1U manufactured by Nihon Seimitsu Kagaku Co., Ltd.
Integrator: Chromato Integrator D-2500 manufactured by Hitachi, Ltd.
Injector: sample injector (model 7725) manufactured by Reodyne Corporation
Column: Senshu Pak DOCOSIL(4.6mm i.d. × 150mm) manufactured by Senshu Scientific Co., Ltd.
Column oven: L-7300 manufactured by Hitachi, Ltd.
Dry reaction tank: Dry Reaction Bath DB-5 manufactured by Takashima Keiki, Ltd.
Analysis software: Chromatocon-PRO manufactured by Runtime Instruments Co., Ltd.

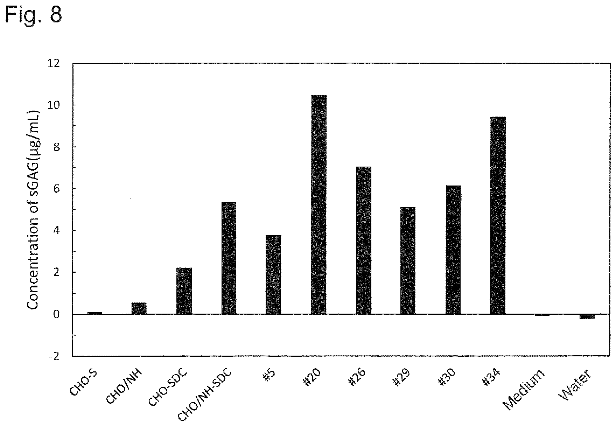

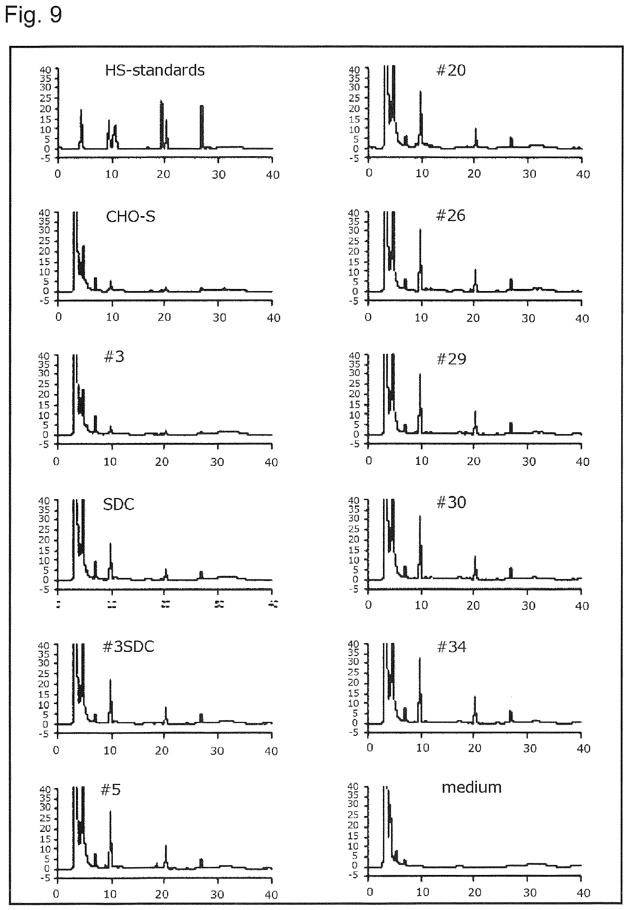

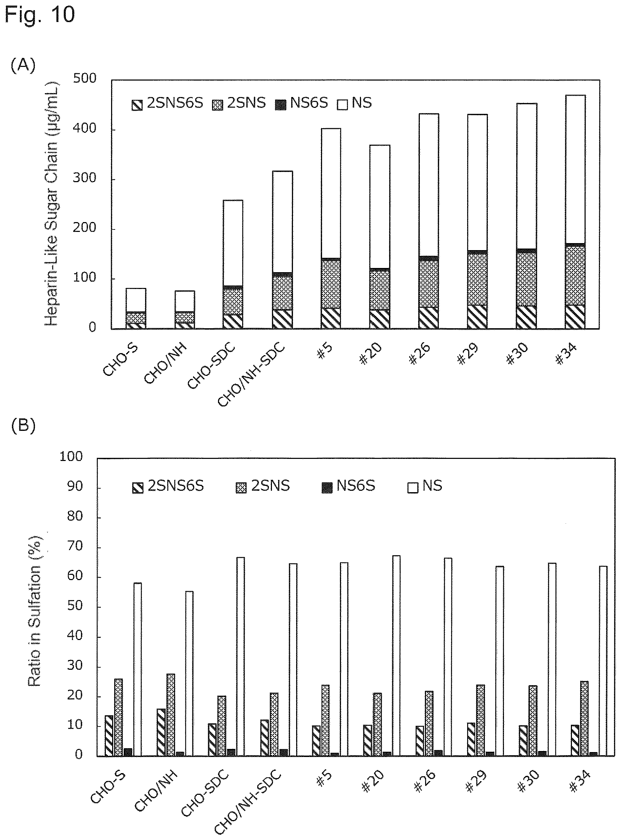

The results of quantifying sGAG in the sample used for the analysis by HPLC are shown in FIG. 8, and chromatograms obtained from standard heparin-derived unsaturated disaccharides (HS-Standards) and the sample are shown in FIG. 9. The results of quantifying a content of heparin-like sugar chains and a ratio of each sugar chain in sulfation by HPLC are shown in FIGS. 10(A) and 10 (B), respectively. Table 1 shows a detected concentration of a sulfated sugar chain (HS) and a ratio of the sulfated sugar chain in the heparin-like substance.

**[Table 1]**

| Sample | Concentration ((µg/mL) | Ratio (%) |
|---|---|---|
| CHO-S | 81 | 23.1 |
| #3 | 76 | 23.3 |
| SDC | 258 | 21.6 |
| #3 SDC | 316 | 22.6 |
| #5 | 402 | 20.1 |
| #20 | 369 | 20.4 |
| #26 | 432 | 23.9 |
| #29 | 431 | 22.8 |
| #30 | 453 | 23.2 |
| #34 | 470 | 24.7 |

As shown in Table 1, a degree of sulfation of a sugar chain detected from samples obtained from the prepared clones (#5, #20, #26, #29, #30, and #34) was about the same as that of heparin, and the production of heparin-like sugar chain was significantly increased as compared with the control (CHO-S, SDC).

### <Measurement of anti-factor Xa activity>

Measurement was performed using a test team heparin S measurement kit manufactured by Sekisui Medical Co., Ltd. (http://www.info.pmda.go.jp/tgo/pack/13A2X00197218081_A_01_10/). The standard heparin was prepared using medical heparin (101 IU/mg). An outline of the measurement principle is as follows.

Constituent Reagent Name and Component:
1. Substrate: N-benzoyl-L-isoleucyl-L-glutamyl (y-OR)-glycyl-L-arginyl-p-nitroanilide hydrochloride (S-2222)
2. Antithrombin III agent: Antithrombin III (derived from human)
3. Factor Xa agent: Factor Xa (derived from bovine)
4. Buffer: 2-amino-2-hydroxymethyl-1,3-propanediol buffer
5. Normal plasma agent: human normal plasma

### Verification of anticoagulation ability

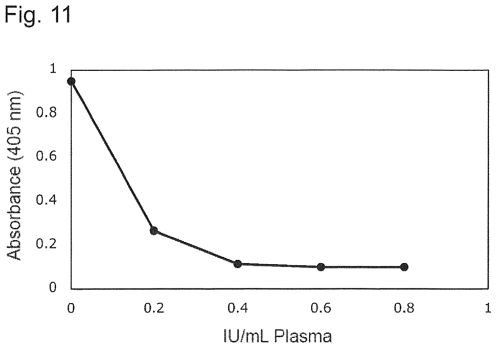

The anticoagulation activity (anti-FXa activity 9) of clones (#20, #26, #29, #30, and #34) having a high degree of sulfation in the above HPLC results was examined using a calibration curve shown in FIG. 11. The results are shown in Table 2.

**[Table 2]**

| Sample | Anti-FXa activity (IU/mL) |
|---|---|
| #20 | 0.09 |
| #26 | 0.02 |
| #29 | 0.1 |
| #30 | 0.09 |
| #34 | 0.09 |

As shown in Table 2, in samples obtained from #20, #29, #30 and #34, the anti-FXa activity of about 0.1 IU per 1 mL of the medium was confirmed. From this result, it was suggested that an antithrombin binding sequence was contained in the heparin-like sugar chain in the culture supernatant by the introduction of NDST2 and Hs3st1.

Although the present invention has been described in detail using specific embodiments, it will be apparent to those skilled in the art that various modifications and variations are possible without departing from the spirit and scope of the present invention. This application is based on a Japanese patent application filed on October 2, 2019 (Application No. 2019-182467), the entire contents thereof being incorporated herein by reference.

## Claims

1. A method for producing a heparin-like substance, the method comprising the following steps (1) to (3):
(1) preparing a mammalian cell that produces a heparin-like substance;
(2) preparing a recombinant cell in which a gene that encodes an extracellular domain of syndecan (hereinafter abbreviated as SDC) is introduced into the mammalian cell that produces a heparin-like substance and is prepared in step (1); and
(3) culturing the recombinant cell prepared in step (2) in a medium, and collecting a heparin-like substance from a resulting culture supernatant.

2. The production method according to claim 1, wherein the mammalian cell that produces a heparin-like substance is a cell into which at least one of a gene that encodes bifunctional heparan sulfate ester N-deacetylase/N-sulfate transferase (hereinafter abbreviated as NDST2) and a gene that encodes heparan sulfate ester glucosamine 3-sulfate transferase 1 (hereinafter abbreviated as Hs3st1) is introduced.

3. The production method according to claim 1 or 2, wherein the mammalian cell is a Chinese hamster-derived ovary (hereinafter abbreviated as CHO) cell.

4. The production method according to claim 2 or 3, wherein NDST2 is a protein comprising any one of the following amino acid sequences (1a) to (1c) and having a function of NDST2:
(1a) the amino acid sequence of SEQ ID NO: 2,
(1b) an amino acid sequence in which one to several amino acids are deleted, substituted or added in the amino acid sequence of SEQ ID NO: 2, and
(1c) an amino acid sequence having 80% or more homology with the amino acid sequence of SEQ ID NO: 2.

5. The production method according to any one of claims 2 to 4, wherein Hs3st1 is a protein comprising any one of the following amino acid sequences (2a) to (2c) and having a function of Hs3st1:
(2a) the amino acid sequence of SEQ ID NO: 3,
(2b) an amino acid sequence in which one to several amino acids are deleted, substituted or added in the amino acid sequence of SEQ ID NO: 3, and
(2c) an amino acid sequence having 80% or more homology with the amino acid sequence of SEQ ID NO: 3.

6. The production method according to any one of claims 1 to 5, wherein the extracellular domain of SDC is a protein comprising any one of the following amino acid sequences (3a) to (3c) and having a function of the extracellular domain of SDC:
(3a) the amino acid sequence of SEQ ID NO: 1,
(3b) an amino acid sequence in which one to several amino acids are deleted, substituted or added in the amino acid sequence of SEQ ID NO: 1, and
(3c) an amino acid sequence having 80% or more homology with the amino acid sequence of SEQ ID NO: 1.

7. The production method according to any one of claims 2 to 6, wherein NDST2 and Hs3st1 are derived from a mouse or a human.

8. The production method according to any one of claims 2 to 7, wherein the gene that encodes NDST2 comprises a base sequence that is any one of the following base sequences (1A) to (1D) and encodes an amino acid sequence of a protein having a function of NDST2:
(1A) the base sequence of SEQ ID NO: 4,
(1B) a base sequence in which one to several bases are deleted, substituted or added in the base sequence of SEQ ID NO: 4,
(1C) a base sequence which hybridizes under stringent conditions with a base sequence complementary to the base sequence of SEQ ID NO: 4, and
(ID) a base sequence having 80% or more homology with the base sequence of SEQ ID NO: 4.

9. The production method according to any one of claims 2 to 8, wherein the gene that encodes Hs3st1 comprises a base sequence that is any one of the following base sequences (2A) to (2D) and encodes an amino acid sequence of a protein having a function of Hs3st1:
(2A) a base sequence of SEQ ID NO: 5,
(2B) a base sequence in which one to several bases are deleted, substituted or added in the base sequence of SEQ ID NO: 5,
(2C) a base sequence which hybridizes under stringent conditions with a base sequence complementary to the base sequence of SEQ ID NO: 5, and
(2D) a base sequence having 80% or more homology with the base sequence of SEQ ID NO: 5.

10. The production method according to any one of claims 1 to 9, wherein the gene that encodes SDC comprises a base sequence that is any one of the following base sequences (3A) to (3D) and encodes an amino acid sequence of a protein having a function of the extracellular domain of SDC:
(3A) a base sequence of SEQ ID NO: 6,
(3B) a base sequence in which one to several amino acids are deleted, substituted or added in the base sequence of SEQ ID NO: 6,
(3C) a base sequence which hybridizes under stringent conditions with a base sequence complementary to the base sequence of SEQ ID NO: 6, and
(3D) a base sequence having 80% or more homology with the base sequence of SEQ ID NO: 6.

11. A recombinant cell in which a gene that encodes an extracellular domain of SDC is introduced into a mammalian cell that produces a heparin-like substance.

12. The recombinant cell according to claim 11, wherein the mammalian cell that produces a heparin-like substance is a cell into which at least one of a gene that encodes NDST2 and a gene that encodes Hs3st1 is introduced.

13. The recombinant cell according to claim 11 or 12, wherein the mammalian cell is a CHO cell.

14. A method for producing a cell that secretes and produces a heparin-like substance, the method comprising: introducing a recombinant expression vector comprising a gene that encodes an extracellular domain of SDC into a mammalian cell that produces a heparin-like substance.
